# EUROPÄISCHE PATENTSCHRIFT

(11) **EP 2 521 518 B1**
(45) Veröffentlichungstag und Bekanntmachung des Hinweises auf die Patenterteilung: **10.08.2016**
(21) Anmeldenummer: 11817469.7
(22) Anmeldetag: 11.10.2011
(51) Int. Cl.: A61F 9/007, A61B 17/32

(54) **VORRICHTUNG ZUM SCHNEIDEN UND ABSAUGEN VON GEWEBE**
APPARATUS FOR CUTTING AND ASPIRATING TISSUE
DISPOSITIF POUR COUPER ET ASPIRER DES TISSUS

(30) Priorität: 05.11.2010 DE 102010050337
(43) Veröffentlichungstag der Anmeldung: 14.11.2012
(73) Patentinhaber: Geuder, Volker, 69126 Heidelberg (DE)
(72) Erfinder: Geuder, Volker, 69126 Heidelberg (DE)
(74) Vertreter: Patent- und Rechtsanwälte Ullrich & Naumann
(86) Internationale Anmeldenummer: PCT/DE2011/050043
(87) Internationale Veröffentlichungsnummer: WO 2012/059092

(56) Entgegenhaltungen:
- WO-A1-98/46147
- DE-A1- 10 032 007
- US-A- 5 106 364
- US-A- 5 474 532
- US-A- 5 980 546

## Beschreibung

Die Erfindung betrifft eine Vorrichtung zum Schneiden und Absaugen von Gewebe aus dem menschlichen oder tierischen Körper, insbesondere zum Einsatz in der Vitrektomie, zum Netzhautpeeling, gemäß der Definition des Gegenstands nach Anspruch 1. Die Vorrichtung umfasst eine äußere Röhre und eine in der äußeren Röhre konzentrisch, mit geringem Spiel alternierend verschiebbare innere Röhre, wobei die äußere Röhre am freien Ende geschlossen ist und nahe dem freien Ende eine seitliche Öffnung mit mindestens einer inneren Schneidkante aufweist. Die innere Röhre ist am freien Ende offen und weist dort eine äußere Schneidkante auf. Die Schneidkanten wirken beim Verschieben der inneren Röhre schneidend zusammen.

Grundsätzlich geht es hier um ein chirurgisches Schneidinstrument zur Entnahme von Gewebe. Mit dem Instrument lässt sich das Gewebe - am bzw. im Körper - schneiden und vom Körper bzw. aus dem Körper heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt wird. Auch lassen sich mit diesem Instrument Blut, Blutgerinnsel und bindegewebsähnliche Veränderungen sowie Bereiche der Netzhaut im Rahmen eines Netzhautpeelings entfernen.

Zum Stand der Technik sei lediglich beispielhaft auf die US 5,630,827 verwiesen, aus der ein für Glaskörperoperationen geeignetes Schneidinstrument bekannt ist. Am proximalen Ende ist das Schneidgerät offen. Am distalen Ende besitzt es eine geschlossene Außenhülse, die in der Nähe des geschlossenen Endes mit mehreren Öffnungen versehen ist. Die Öffnungen bilden jeweils einen Schlitz und ermöglichen das Eintreten von Glaskörpergewebe in das Lumen der Außenhülse. Außerdem besitzt das Schneidinstrument eine konzentrisch zur äußeren Hülse angeordnete innere Hülse, die in der äußeren Hülse längsbeweglich aufgenommen ist und an ihrem distalen Ende eine scharfe Kante zum Schneiden des Glaskörpergewebes beim Vorschieben der Innenhülse umfasst.

Bei der aus der US 5,630,827 bekannten Vorrichtung ist wesentlich, dass die innere Röhre an ihrem freien vorderen Ende eine offene umlaufende Kante aufweist, die als Schneidkante dient. Entsprechend ist diese Kante geformt bzw. geschliffen. Eine schneidende Wirkung kommt dem äußeren Randbereich zu, so dass hier fortan von einer äußeren Schneidkante der inneren Röhre die Rede ist. Diese äußere Schneidkante wirkt mit einer oder mehreren inneren Schneidkanten der äußeren Röhre zusammen, so dass in die Vorrichtung eingedrungenes Gewebe beim gegenseitigen Vorbeigleiten der Schneidkanten geschnitten bzw. abgetrennt wird. Danach kann das Gewebe aus dem Inneren der Vorrichtung, genauer gesagt durch die innere Röhre hindurch, abgesaugt werden.

Aus der WO 98/52502 ist ebenfalls ein gattungsbildendes Schneidinstrument zur Entnahme von Glaskörpergewebe bekannt. Am distalen Ende des Instruments ist eine geschlossene Außenhülse vorgesehen, die in der Nähe des distalen Endes mehrere Schlitze aufweist, durch die Glaskörpergewebe eindringen kann. Konzentrisch zur Außenhülse ist eine längsbewegliche Innenhülse vorgesehen, die an ihrem distalen Ende eine scharfe Kante zum Schneiden des eingedrungenen Glaskörpergewebes besitzt. Außerdem ist ein Antriebsmechanismus für die Innenhülse vorgesehen, durch den die Innenhülse mit ihrer Schneidkante im Bereich des distalen Endes über die in der Außenhülse angeordneten Schlitze hinweg bewegbar ist, so dass dort jeweils ein Schneidvorgang stattfindet.

Aus der US 5,106,364 ist eine Vorrichtung zum Schneiden gemäß dem Oberbegriff des Anspruchs 1 bekannt.

Die bekannten Vorrichtungen der gattungsbildenden Art sind jedoch in der Praxis in zweierlei Hinsicht problematisch. Zum einen ist die Arbeitsgeschwindigkeit durch die Frequenz definiert, mit der die innere Röhre hin und her bewegt wird, nämlich mit ihrer äußeren Schneidkante an der oder den inneren Schneidkanten der äußeren Röhre vorbei. So lässt sich mit jedem Hub der inneren Röhre nur eine gewisse Menge an Gewebe schneiden, wodurch der Durchsatz durch die maximale Frequenz begrenzt ist.

Außerdem ist nachteilig, dass die bekannte Vorrichtung ganz erhebliche Druck- bzw. Unterdruckschwankungen im Auge vermittelt, nämlich aufgrund der Tatsache, dass die zum Absaugen dienende Öffnung oder Öffnungen mal komplett geschlossen und mal komplett geöffnet ist/sind, je nach Position der inneren Röhre. Folglich entsteht im Körper bzw. Auge ein alternierender Unterdruck, der sich kaum kompensieren lässt. Die so hervorgerufenen - erheblichen - Druckschwankungen können zu einer Schädigung des Auges führen.

Der vorliegenden Erfindung liegt daher die Aufgabe zugrunde, die gattungsbildende Vorrichtung zum Schneiden und Absaugen von Gewebe aus dem menschlichen oder tierischen Körper, insbesondere zum Einsatz in der Vitrektomie, derart auszugestalten und weiterzubilden, dass sich eine erhebliche Schneidleistung und somit ein hoher Durchsatz realisieren lässt, ohne die Frequenz für die Hin- und Herbewegung der inneren Röhre zu erhöhen und dies möglichst unter Vermeidung von Druckschwankungen im Körper.

Die voranstehende Aufgabe ist durch die Merkmale des Patentanspruchs 1 gelöst. Danach ist die gattungsbildende Vorrichtung dadurch gekennzeichnet, dass die innere Röhre, nahe dem freien Ende, mindestens eine seitliche Öffnung mit mindestens einer weiteren äußeren Schneidkante aufweist.

Erfindungsgemäß ist erkannt worden, dass es in Bezug auf die Schneidleistung von ganz erheblichem Vorteil ist, wenn die innere Röhre mindestens eine weitere Schneidkante aufweist, die mit der Schneidkante oder den Schneidkanten der äußeren Röhre zusammenwirkt. Dies lässt sich auf verblüffend einfache Weise dadurch realisieren, dass die innere Röhre, nahe dem freien Ende, mindestens eine seitliche Öffnung aufweist, ähnlich wie die äußere Röhre. Diese seitliche Öffnung in der inneren Röhre umfasst mindestens eine weitere äußere Schneidkante, so dass die innere Röhre insgesamt mit mindestens zwei Schneidkanten ausgestattet ist. Im Verlaufe des Hubs der inneren Röhre bewegt sich somit deren vordere Schneidkante, d. h. die Schneidkante am freien Ende der inneren Röhre und die mindestens eine weitere Schneidkante, an der inneren Schneidkante der äußeren Röhre vorbei, so dass unter Zugrundelegung der zuvor erörterten Konstruktion pro Hub mindestens zweimal geschnitten wird.

Geht man davon aus, dass die seitliche Öffnung der inneren Röhre, beispielsweise in Form eines Schlitzes, der im Sinne eines Teilringsegments ausgebildet ist, mit zwei zusätzlichen äußeren Schneidkanten ausgestattet ist, kann die innere Röhre zwei äußere Schneidkanten in der seitlichen Öffnung und die vordere Schneidkante umfassen, d. h. insgesamt drei äußere Schneidkanten, so dass bei einer Hin- und Herbewegung der inneren Röhre dreimal geschnitten wird, nämlich zweimal bei der Vorwärtsbewegung und mindestens einmal bei einer rückwärtigen Bewegung der inneren Röhre.

In erfindungsgemäßer Weise ist jedenfalls wesentlich, dass die innere Röhre, nahe dem freien Ende, mindestens eine, durchaus aber auch zwei oder mehrere seitliche Öffnungen aufweisen kann, die insgesamt mit drei oder vier äußeren Schneidkanten ausgestattet sind. Im Falle von drei Öffnungen können diese mit bis zu sechs zusätzlichen Schneidkanten ausgestattet sein.

In weiter vorteilhafter Weise sind die Öffnungen in der inneren Röhre als parallel zueinander ausgebildete Schlitze mit parallelen, einander gegenüberliegenden Schneidkanten ausgeführt. Beliebige andere Ausgestaltungsformen, beispielsweise ovale Öffnungen mit entsprechend gebogenen Schneidkanten, sind denkbar.

Die Schneidleistung lässt sich abermals erhöhen, nämlich durch eine weitere konstruktive Maßnahme, wonach die äußere Röhre, nahe dem freien, geschlossenen Ende, zwei benachbarte Öffnungen aufweist, die insgesamt mit drei oder vier inneren Schneidkanten ausgestattet sind. Diese Schneidkanten wirken mit den zusätzlichen Schneidkanten der inneren Röhre zusammen, so dass sich insgesamt mehrere Schneidvorgänge ergeben, je nach Anzahl der aneinander vorbei gleitenden Schneidkanten, und zwar mit jedem Teilhub.

Die in der äußeren Röhre ausgebildeten Öffnungen können, wie die Öffnungen in der inneren Röhre, als parallel zueinander ausgebildete Schlitze mit parallelen Schneidkanten ausgeführt sein. Auch hier ist es denkbar, die Öffnungen unterschiedlich groß und mit unterschiedlichen Formen vorzusehen, beispielsweise linsenförmig bzw. oval. Entsprechend können die Schneidkanten umlaufend ausgebildet sein, so dass sie in Bezug auf den Vorwärtshub und auf den Rückwärtshub wie zwei unterschiedlich ausgerichtete Schneidkanten wirken.

Grundsätzlich ist es denkbar, dass die Schneidkanten quer zur Längsachse der Röhren verlaufen. Ebenso ist es denkbar, dass die Schneidkanten schräg zur Längsachse der Röhren verlaufen oder gar in Längsrichtung versetzt zueinander angeordnet sind.

Im Rahmen einer weiteren Variante ist es denkbar, dass die Schneidkanten der inneren Röhre einen anderen Winkel zur Längsachse haben als die Schneidkanten der äußeren Röhre. Insoweit ergibt sich bei der Hubbewegung der inneren Röhre eine Art Schereneffekt in Bezug auf die aneinander vorbei gleitenden Schneidkanten. Dies begünstigt die Schneidleistung abermals.

Grundsätzlich können die Schneidkanten geradlinig, gebogen, gewellt oder sogar abgewinkelt ausgeführt sein, um nämlich in Bezug auf das zu schneidende Gewebe eine besondere Schneidwirkung zu erzielen.

Auch ist es denkbar, dass die Öffnungen, die sich in die jeweilige Röhre nach innen erstrecken, orthogonal zur Längsachse der Röhren verlaufen. Auch ist es möglich, dass die Öffnungen bzw. Schlitze zum freien Ende der Röhre hin oder vom freien Ende der Röhre weg nach innen geneigt sind, d. h. unter einem von 90° abweichenden Winkel.

Wie bereits zuvor erwähnt, verfügt die äußere Röhre über innere Schneidkanten und die innere Röhre über äußere Schneidkanten, wobei die jeweiligen Schneidkanten bei der Hubbewegung der inneren Röhre aneinander vorbei gleiten, wodurch sich sowohl beim Vorwärtshub als auch beim Rückwärtshub die Schneidwirkung ergibt. Von den Schneidkanten aus erstrecken sich durch die Materialstärke der Röhren definierte Schneidflächen, und zwar jeweils von der jeweiligen Schneidkante abfallend nach außen (bei der äußeren Röhre) und nach innen (bei der inneren Röhre). So lässt sich die jeweilige Schneidkante über die Schneidfläche definieren und auch ggf. auch schärfen bzw. nacharbeiten.

Die Öffnungen bzw. Schlitze der jeweiligen Röhren sind grundsätzlich axial voneinander beabstandet. Auch ist es denkbar, dass mehrere Öffnungen oder Schlitze in Umfangsrichtung versetzt zueinander angeordnet sind, um nämlich in Umfangsrichtung der Vorrichtung eine Schneidwirkung zu erzeugen. Entsprechend dem Bedarf lassen sich unterschiedlichste Anordnungen, auch Verschachtelungen von Öffnungen, über den Umfang der jeweiligen Röhre hinweg realisieren.

In Bezug auf die Vermeidung von Druckschwankungen im Körper ist es von ganz besonderem Vorteil, wenn die Vorrichtung durch eine einfache konstruktive und steuerungstechnische Maßnahme derart ausgelegt ist, dass im Betrieb im jeweiligen Körper keinerlei Druckschwankungen auftreten, wenngleich jede Öffnung für sich gesehen durch die innere Röhre unterschiedlich abgedeckt ist.

Erfindungsgemäß sind die Öffnungen und somit die Schneidkanten der Röhren über den gesamten Bewegungsablauf der inneren Röhre derart zueinander angeordnet sind, dass die Öffnungen derart dimensioniert sind und dass die Bewegung der inneren Röhre derart begrenzt bzw. geregelt ist, dass zu jedem Zeitpunkt des Bewegungsablaufs, d. h. über den gesamten Vorwärtshub und Rückwärtshub der inneren Röhre hinweg, der gleiche Strömungsquerschnitt in die innere Röhre hinein besteht. Dies bedeutet, dass beispielsweise eine vordere Öffnung in der äußeren Röhre komplett geöffnet ist, während eine zweite hintere Öffnung in der äußeren Röhre komplett durch die innere Röhre geschlossen ist. Beim Rückwärtshub der inneren Röhre wird die vordere Öffnung allmählich geschlossen, nämlich um das gleiche Maß, wie die hintere Öffnung der äußeren Röhre durch den Rückwärtshub der inneren Röhre freigegeben wird. Ungeachtet der Anzahl der Öffnungen ist es bei entsprechender Auslegung möglich, die Anordnung von äußerer Röhre und innerer Röhre und der dort vorgesehenen Öffnungen bzw. Schlitze derart auszulegen, dass der voranstehenden Vorschrift jederzeit entsprochen wird, dass nämlich zu jedem Zeitpunkt des Bewegungsablaufs, d. h. über den gesamten Vorwärtshub und Rückwärtshub der inneren Röhre hinweg, der wirksame Strömungsquerschnitt durch alle Öffnungen hindurch der gleiche ist, so dass über den Bewegungsablauf hinweg keinerlei Druckschwankungen aufgrund des im Inneren der Vorrichtung herrschenden, zum Absaugen erforderlichen Unterdrucks erzeugt wird. Ganz im Gegenteil bleibt der nach außen wirkende Sog bzw. Unterdruck stets der gleiche, so dass eine aufgrund von Druckschwankungen mögliche Schädigung des Auges ausgeschlossen ist, nämlich unter Vermeidung entsprechender Druckschwankungen, die nach außen wirken.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1-6: in schematischen Ansichten, teilweise, ein Ausführungsbeispiel einer erfindungsgemäßen Vorrichtung zum Schneiden und Absaugen von Gewebe, wobei die Figuren den Bewegungsablauf der inneren Röhre in der äußeren Röhre und die dort jeweils vorgesehenen Öffnungen und Schneidkanten zeigen und
- Fig. 7: in einer schematischen Draufsicht eine Variante der erfindungsgemäßen Vorrichtung, wonach der in die innere Röhre hinein gerichtete Strömungspfad zu jedem Zeitpunkt des Bewegungsablaufs konstant ist.

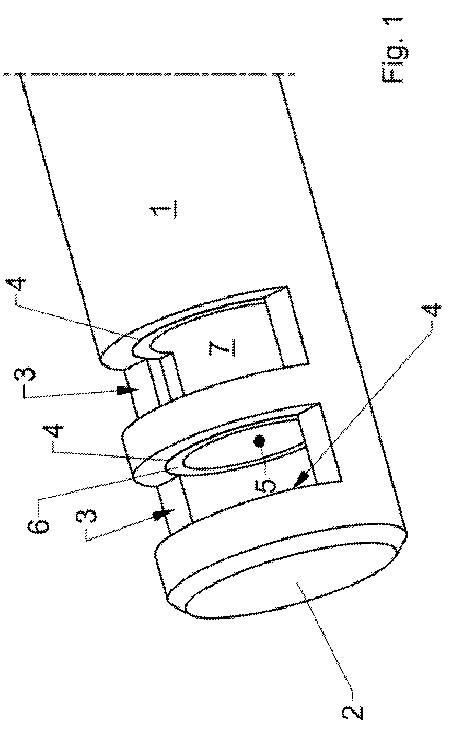

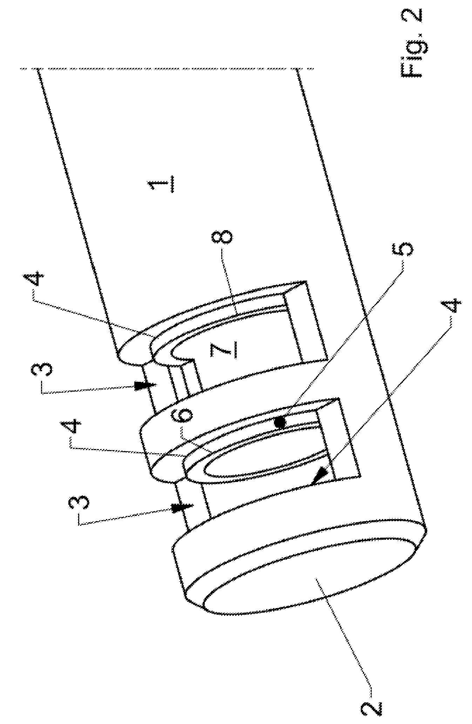

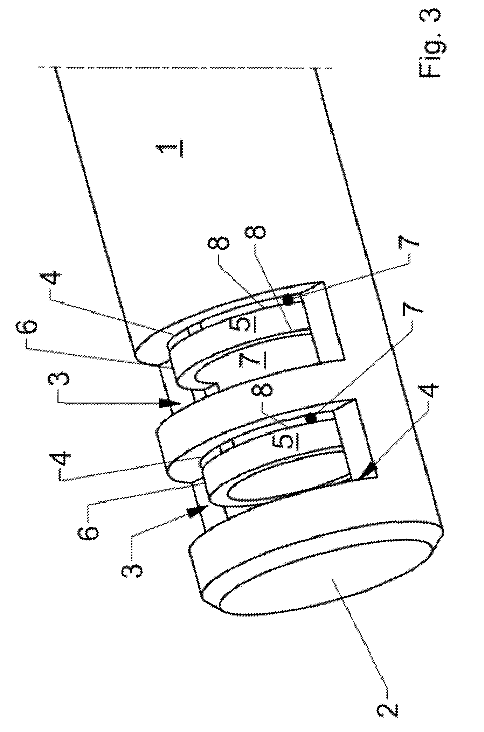

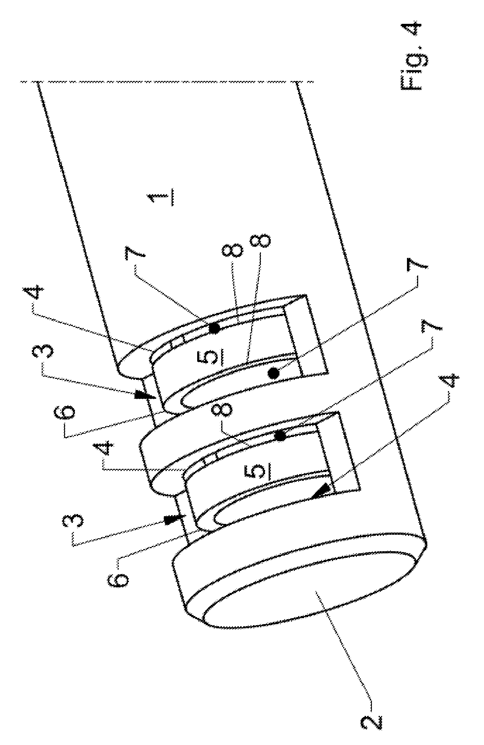

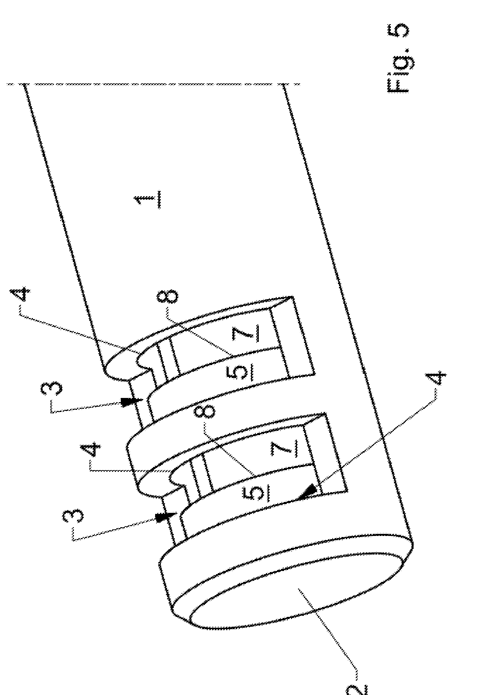

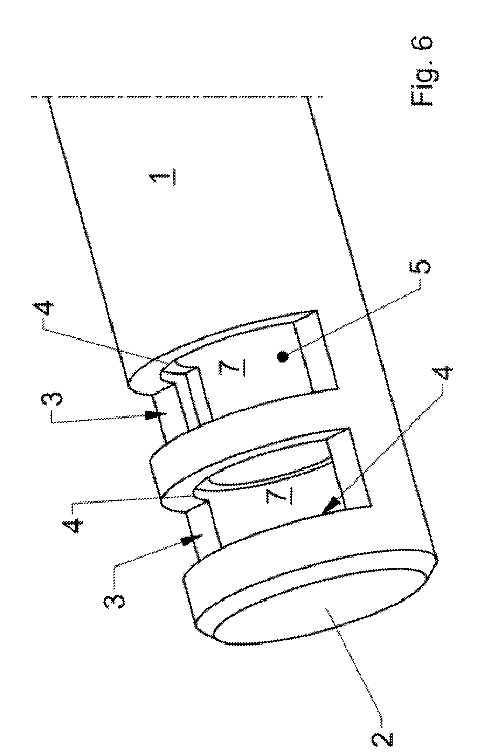

Die Fig. 1 bis 6 zeigen in schematischen Ansichten, stark vergrößert, den Arbeitsbereich der erfindungsgemäßen Vorrichtung, wobei es sich hier um einen sogenannten Vitrektor zum Einsatz in der Vitrektomie handelt, nämlich um eine Vorrichtung zur Glaskörperentfernung aus dem menschlichen Auge.

Die Figuren 1 bis 6 zeigen gemeinsam den Bewegungsablauf und dabei die jeweilige Stellung der beweglichen Teile zueinander.

Im Konkreten zeigen die Figuren 1 bis 6 - teilweise - die äußere Röhre 1, die am freien Ende 2 geschlossen ist. Bei dem in den Figuren 1 bis 6 gezeigten Ausführungsbeispiel ist die äußere Röhre 1 mit insgesamt zwei Öffnungen 3 ausgestattet, die in Form symmetrischer Schlitze ausgebildet sind. Aufgrund der Vorkehrung der beiden Öffnungen 3 verfügt die äußere Röhre 1 über insgesamt vier Schneidkanten 4.

In der äußeren Röhre 1 ist konzentrisch, mit geringem Spiel alternierend verschiebbar, eine innere Röhre 5 angeordnet. Die Figuren 1 bis 6 lassen erkennen, dass die innere Röhre 5 an ihrem vorderen freien Ende offen ist. Entsprechend ist dort eine erste Schneidkante 6 ausgebildet. Je nach Auslegung des Hubs der inneren Röhre 5 schneidet die Schneidkante 6 jedes Mal dann, wenn sie an einer gegen die Bewegungsrichtung der inneren Röhre 5 gerichtete Schneidkante 4 vorbei gleitet. Bei dem in den Figuren 1 bis 6 gezeigten Ausführungsbeispiel schneidet daher die Schneidkante 6 maximal zweimal, wenn sie nämlich bei entsprechend ausgelegtem Hub zweimal an einer jeweils entgegengerichteten Schneidkante 4 der äußeren Röhre 1 vorbei geführt wird.

Nun kann die innere Röhre 5 mit einer oder mit zwei Öffnungen 7 ausgestattet sein, die ebenfalls in Form eines Spalts ausgeführt sind. Die Öffnungen 7 der inneren Röhre 5 können in etwa das gleiche Öffnungsmaß wie die Öffnungen 3 der äußeren Röhre 1 haben.

Bei Vorkehrung einer Öffnung 7 kann diese zwei zusätzliche äußere Schneidkanten 8 aufweisen, die beim Vorbeigleiten an entgegengesetzt gerichteten Schneidkanten 4 der äußeren Röhre 1 einen Schneidvorgang bewirken.

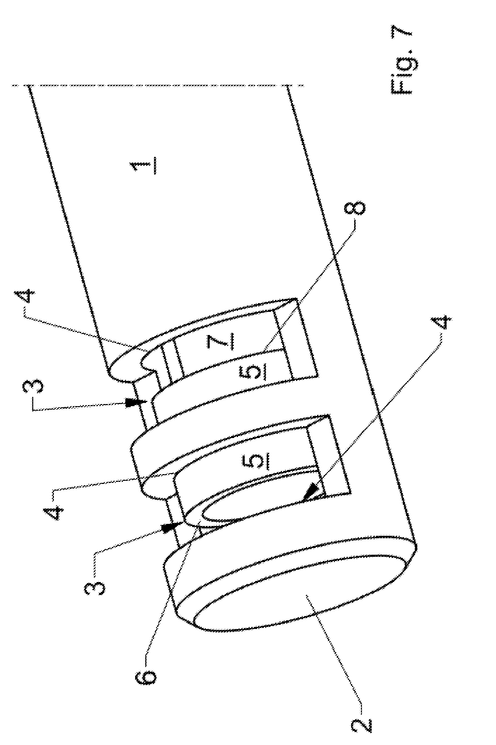

Sowohl bei Vorkehrung einer einzigen Öffnung 7 in der inneren Röhre 5 als auch bei Vorkehrung von zwei Öffnungen 7 der innere Röhre 5 können diese derart dimensioniert und positioniert sein, dass bei geeigneter Bewegung der inneren Röhre 5 innerhalb der äußeren Röhre 1 stets der gleiche Strömungsquerschnitt wirkt, nämlich dann, wenn die eine Öffnung 3 in der äußeren Röhre 1 geöffnet oder geschlossen wird, und die andere Öffnung 3 in der äußeren Röhre 1 geschlossen oder geöffnet wird. Insoweit sei auf die schematische Darstellung von Fig. 7 verwiesen. In einer Mittelposition wären somit die Öffnungen 3 in der äußeren Röhre 1 jeweils hälftig durch die Wandung der inneren Röhre 5 geschlossen bzw. entsprechend der Position der Schneidkanten 8 der inneren Röhre 5 geöffnet.

An dieser Stelle sei angemerkt, dass die erfindungsgemäße Vorrichtung in den Figuren lediglich schematisch dargestellt ist, insbesondere um grundsätzliche Ausgestaltungen und den Bewegungsablauf zu zeigen. Auf eine Darstellung von Details wurde der Klarheit wegen verzichtet.

Schließlich sei angemerkt, dass das voranstehend erörterte Ausführungsbeispiel lediglich der beispielhaften Erörterung der beanspruchten Lehre dient, diese jedoch nicht auf das Einführungsbeispiel einschränkt.

### Bezugszeichenliste

- 1: äußere Röhre
- 2: freies Ende von 1
- 3: seitliche Öffnung von 1
- 4: innere Schneidkanten von 1
- 5: innere Röhre
- 6: äußere Schneidkante von 5
- 7: seitlichen Öffnungen von 5
- 8: zusätzliche äußere Schneidkanten von 5

## Patentansprüche

1. Vorrichtung zum Schneiden und Absaugen von Gewebe aus dem menschlichen oder tierischen Körper, insbesondere zum Einsatz in der Vitrektomie, zum Netzhauptpeeling, mit einer äußeren Röhre (1) und einer in der äußeren Röhre (1) konzentrisch, mit geringem Spiel alternierend verschiebbaren inneren Röhre (5), wobei die äußere Röhre (1) am freien Ende geschlossen ist und nahe dem freien Ende (2) eine seitliche Öffnung (3) mit mindestens einer inneren Schneidkante (6) aufweist, wobei die innere Röhre (5) am freien Ende (2) offen ist und dort eine äußere Schneidkante (6) aufweist und wobei die Schneidkanten (4, 6) beim Verschieben der inneren Röhre (5) schneidend zusammenwirken, wobei die innere Röhre (5), nahe dem freien Ende (2), mindestens eine seitliche Öffnung (7) mit mindestens einer weiteren äußeren Schneidkante (8) aufweist,
**dadurch gekennzeichnet, dass** die Öffnungen (3, 7) und somit die Schneidkanten (4, 6, 8) der Röhren (1, 5) über den gesamten Bewegungsablauf der inneren Röhre (5) derart zueinander angeordnet, die Öffnungen (3, 7) derart dimensioniert und die Bewegung der inneren Röhre (5) derart begrenzt sind, dass zu jedem Zeitpunkt des Bewegungsablaufs der gleiche Strömungsquerschnitt in die innere Röhre (5) hinein besteht.

2. Vorrichtung nach Anspruch 1, **dadurch gekennzeichnet, dass** die seitliche Öffnung (7) der inneren Röhre (5) mit zwei äußeren Schneidkanten (8) ausgestattet ist.

3. Vorrichtung nach Anspruch 1 oder 2, **dadurch gekennzeichnet, dass** die innere Röhre (5), nahe dem freien Ende, zwei seitliche Öffnungen (7) aufweist, die insgesamt mit drei oder vier äußeren Schneidkanten (8) ausgestattet sind.

4. Vorrichtung nach Anspruch 3, **dadurch gekennzeichnet, dass** die Öffnungen (7) als parallel zueinander ausgebildete Schlitze mit parallelen Schneidkanten (8) ausgeführt sind.

5. Vorrichtung nach einem der Ansprüche 1 bis 4, **dadurch gekennzeichnet, dass** die äußere Röhre (1), nahe dem freien, geschlossenen Ende (2), zwei benachbarte Öffnungen (3) aufweist, die insgesamt mit drei oder vier inneren Schneidkanten (4) ausgestattet sind.

6. Vorrichtung nach einem der Ansprüche 1 bis 5, **dadurch gekennzeichnet, dass** die Öffnungen (3, 7) als parallel zueinander ausgebildete Schlitze mit parallelen Schneidkanten (4, 8) ausgeführt sind.

7. Vorrichtung nach einem der Ansprüche 1 bis 6, **dadurch gekennzeichnet, dass** die Schneidkanten (4, 6) quer zur Längsachse der Röhren (4, 5) verlaufen.

8. Vorrichtung nach einem der Ansprüche 1 bis 6, **dadurch gekennzeichnet, dass** die Schneidkanten (4, 6) schräg zur Längsachse der Röhren (1, 5) verlaufen.

9. Vorrichtung nach einem der Ansprüche 1 bis 8, **dadurch gekennzeichnet, dass** die Schneidkanten (6, 8) der inneren Röhre (5) einen anderen Winkel zur Längsachse haben als die Schneidkanten (4) der äußeren Röhre (1).

10. Vorrichtung nach einem der Ansprüche 1 bis 9, **dadurch gekennzeichnet, dass** die Schneidkanten (4, 6, 8) geradlinig ausgeführt sind.

11. Vorrichtung nach einem der Ansprüche 1 bis 9, **dadurch gekennzeichnet, dass** die Schneidkanten (4, 6, 8) gebogen, gewellt oder abgekantet ausgeführt sind.

12. Vorrichtung nach einem der Ansprüche 1 bis 11, **dadurch gekennzeichnet, dass** sich von den Schneidkanten (4, 6, 8) aus durch die Materialstärke der Röhren (1, 5) definierte Schneidflächen jeweils von der Schneidkante (4, 6, 8) abfallend nach außen (bei der äußeren Röhre (1)) und nach innen (bei der inneren Röhre (5) erstrecken.

13. Vorrichtung nach einem der Ansprüche 1 bis 12, **dadurch gekennzeichnet, dass** die Öffnungen (3, 7) bzw. Schlitze axial voneinander beabstandet sind.

14. Vorrichtung nach einem der Ansprüche 1 bis 12, **dadurch gekennzeichnet, dass** die Öffnungen (3, 7) oder Schlitze in Umfangsrichtung versetzt zueinander angeordnet sind.

## Claims

1. Device for cutting and drawing tissue from the human or animal body, in particular for use in vitrectomy, for retina peeling, having an outer tube (1) and an inner tube (5) which can be displaced in the outer tube (1) in an alternating, concentric manner with little play, wherein the outer tube (1) is closed at the free end and close to the free end (2) has a lateral opening (3) with at least one inner cutting edge (6), wherein the inner tube (5) is open at the free end (2) and has at that location an outer cutting edge (6), and wherein the cutting edges (4, 6) cooperate in a cutting manner when the inner tube (5) is displaced, wherein the inner tube (5) close to the free end (2) has at least one lateral opening (7) with at least one additional outer cutting edge (8), **characterised in that** the openings (3, 7) and consequently the cutting edges (4, 6, 8) of the tubes (1, 5) are arranged with respect to each other over the entire movement sequence of the inner tube (5) in such a manner, the openings (3, 7) are sized in such a manner and the movement of the inner tube (5) is limited in such a manner that at all times of the movement sequence the same cross-section of flow into the inner tube (5) exists.

2. Device according to claim 1, **characterised in that** the lateral opening (7) of the inner tube (5) is provided with two outer cutting edges (8).

3. Device according to claim 1 or claim 2, **characterised in that** the inner tube (5) close to the free end has two lateral openings (7) which are provided with a total of three or four outer cutting edges (8).

4. Device according to claim 3, **characterised in that** the openings (7) are constructed as mutually parallel slots with parallel cutting edges (8).

5. Device according to any one of claims 1 to 4, **characterised in that** the outer tube (1) close to the free closed end (2) has two adjacent openings (3) which are provided with a total of three or four inner cutting edges (4).

6. Device according to any one of claims 1 to 5, **characterised in that** the openings (3, 7) are constructed as mutually parallel slots with parallel cutting edges (4, 8).

7. Device according to any one of claims 1 to 6, **characterised in that** the cutting edges (4, 6) extend transversely relative to the longitudinal axis of the tubes (4, 5).

8. Device according to any one of claims 1 to 6, **characterised in that** the cutting edges (4, 6) extend in an oblique manner relative to the longitudinal axis of the tubes (1, 5).

9. Device according to any one of claims 1 to 8, **characterised in that** the cutting edges (6, 8) of the inner tube (5) have a different angle with respect to the longitudinal axis from that of the cutting edges (4) of the outer tube (1).

10. Device according to any one of claims 1 to 9, **characterised in that** the cutting edges (4, 6, 8) are constructed in a rectilinear manner.

11. Device according to any one of claims 1 to 9, **characterised in that** the cutting edges (4, 6, 8) are constructed in a bent, undulating or bevelled manner.

12. Device according to any one of claims 1 to 11, **characterised in that** cutting faces defined from the cutting edges (4, 6, 8) through the material thickness of the tubes (1, 5) extend from the cutting edge (4, 6, 8) in a manner descending outwards (in the case of the outer tube (1)) and inwards (in the case of the inner tube (5)).

13. Device according to any one of claims 1 to 12, **characterised in that** the openings (3, 7) or slots are axially spaced apart from each other.

14. Device according to any one of claims 1 to 12, **characterised in that** the openings (3, 7) or slots are arranged offset with respect to each other in the peripheral direction.

## Revendications

1. Dispositif de coupe et d'aspiration de tissu du corps humain ou animal, en particulier destiné à être utilisé en vitrectomie, pour le peeling de la rétine, avec un tube extérieur (1) et un tube intérieur (5) pouvant coulisser de façon concentrique dans le tube extérieur (1) de façon alternée avec un faible jeu, le tube extérieur (1) étant fermé à l'extrémité libre et présentant près de l'extrémité libre (2) une ouverture (3) latérale avec au moins une arête de coupe (6) intérieure, le tube intérieur (5) étant ouvert à l'extrémité libre (2) et présentant à cet endroit une arête de coupe (6) extérieure, et les arêtes de coupe (4, 6) coopérant pour la coupe lors du coulissement du tube intérieur (5), le tube intérieur (5) présentant, près de l'extrémité libre (2), au moins une ouverture (7) latérale avec au moins une autre arête de coupe (8) extérieure,
**caractérisé en ce que** les ouvertures (3, 7) et donc les arêtes de coupe (4, 6, 8) des tubes (1, 5) sont disposées de façon juxtaposée entre elles pendant tout le processus de mouvement du tube intérieur (5) de telle sorte, les ouvertures (3, 7) sont dimensionnées de telle sorte et le mouvement du tube intérieur (5) est limité de telle sorte qu'à chaque instant du processus de mouvement, il y a la même section transversale d'écoulement dans l'intérieur du tube intérieur (5).

2. Dispositif selon la revendication 1, **caractérisé en ce que** l'ouverture (7) latérale du tube intérieur (5) est équipée de deux arêtes de coupe (8) extérieures.

3. Dispositif selon la revendication 1 ou 2, **caractérisé en ce que**, près de l'extrémité libre, le tube intérieur (5) présente deux ouvertures (7) latérales qui sont équipées au total de trois ou quatre arêtes de coupe (8) extérieures.

4. Dispositif selon la revendication 3, **caractérisé en ce que** les ouvertures (7) sont réalisées sous forme de fentes constituées de façon parallèle entre elles avec des arêtes de coupe (8) parallèles.

5. Dispositif selon l'une des revendications 1 à 4, **caractérisé en ce que**, près de l'extrémité libre (2) fermée, le tube extérieur (1) présente deux ouvertures (3) voisines qui sont équipées au total de trois ou quatre arêtes de coupe (4) intérieures.

6. Dispositif selon l'une des revendications 1 à 5, **caractérisé en ce que** les ouvertures (3, 7) sont réalisées sous forme de fentes constituées de façon parallèle entre elles avec des arêtes de coupe (4, 8) parallèles.

7. Dispositif selon l'une des revendications 1 à 6, **caractérisé en ce que** les arêtes de coupe (4, 6) sont transversales par rapport à l'axe longitudinal des tubes (4, 5).

8. Dispositif selon l'une des revendications 1 à 6, **caractérisé en ce que** les arêtes de coupe (4, 6) sont obliques par rapport à l'axe longitudinal des tubes (1, 5).

9. Dispositif selon l'une des revendications 1 à 8, **caractérisé en ce que** les arêtes de coupe (6, 8) du tube intérieur (5) ont, par rapport à l'axe longitudinal, un autre angle que les arêtes de coupe (4) du tube extérieur (1).

10. Dispositif selon l'une des revendications 1 à 9, **caractérisé en ce que** les arêtes de coupe (4, 6, 8) sont réalisées de façon rectiligne.

11. Dispositif selon l'une des revendications 1 à 9, **caractérisé en ce que** les arêtes de coupe (4, 6, 8) sont réalisées de façon courbée, ondulée ou repliée.

12. Dispositif selon l'une des revendications 1 à 11, **caractérisé en ce que** des surfaces de coupe définies à partir des arêtes de coupe (4, 6, 8) par l'épaisseur de matériau des tubes (1, 5) s'étendent respectivement de façon inclinée à partir de respectivement l'arête de coupe (4, 6, 8) vers l'extérieur (pour le tube extérieur (1)) et vers l'intérieur (pour le tube intérieur (5)).

13. Dispositif selon l'une des revendications 1 à 12, **caractérisé en ce que** les ouvertures (3, 7) ou respectivement les fentes sont espacées axialement les unes des autres.

14. Dispositif selon l'une des revendications 1 à 12, **caractérisé en ce que** les ouvertures (3, 7) ou respectivement les fentes sont disposées de façon décalée les unes par rapport aux autres dans la direction circonférentielle.
